Europäisches Patentamt

European Patent Office

Office européen des brevets

(19)

(11) Numéro de publication : **0 488 906 A1**

## (12) DEMANDE DE BREVET EUROPEEN

(21) Numéro de dépôt : **91420379.9**

(22) Date de dépôt : **24.10.91**

(51) Int. Cl.$^5$ : **A61B 17/064,** A61B 17/58

Une requête en rectification à savoir l'addition de la Belgique dans la liste des pays désignés a été présentée conformémentà la règle 88 CBE.

(30) Priorité : **30.10.90 FR 9013684**

(43) Date de publication de la demande :
**03.06.92 Bulletin 92/23**

(84) Etats contractants désignés :
**BE DE ES FR IT**

(71) Demandeur : **Mai, Christian**
**74 boulevard des Belges**
**F-69006 Lyon (FR)**

(72) Inventeur : **Mai, Christian**
**74 boulevard des Belges**
**F-69006 Lyon (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

(54) **Agraphe et plaque d'osteosynthèse à compression dynamique auto-rétentive.**

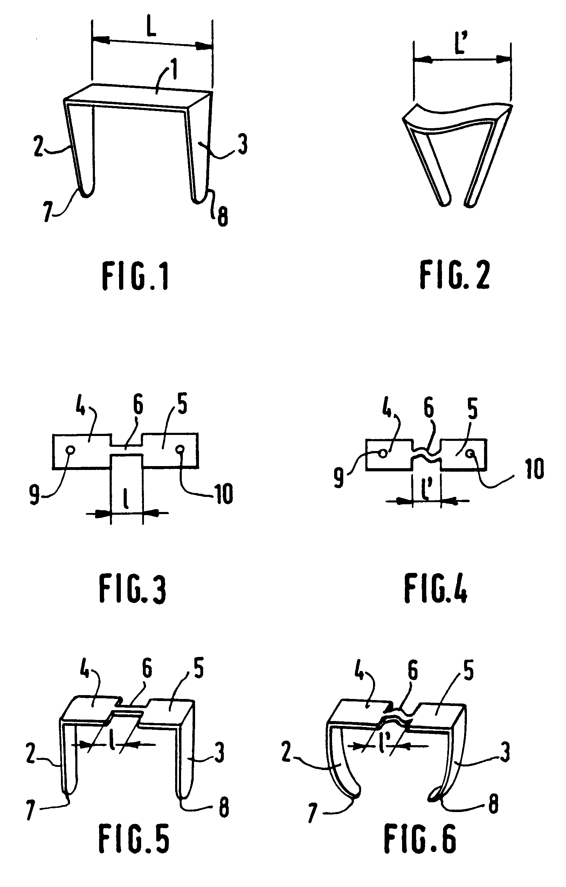

(57) Plaque d'ostéosynthèse, réalisée en un alliage martensitique thermo-élastique, dont les températures de transformation sont :
— température de transformation martensitique Ms inférieure à 10°C ;
— température de transformation austénitique As supérieure à 15°C ;
le passage de la température martensitique à la température austénitique induisant un raccourcissement de la longueur de la plaque,
**caractérisée** en ce qu'elle est éduquée pour se présenter sous forme rectiligne (fig.1) à une température inférieure à la température de transformation martensitique Ms du matériau qui la compose, et sous forme ondulée (fig.2) à une température supérieure à la température de transformation austénitique As dudit matériau.

FIG.1

FIG.2

EP 0 488 906 A1

L'invention concerne un nouveau type d'agrafe et de plaque d'ostéosynthèse présentant des caractéristiques de compression dynamique et d'auto-rétension.

Par "compression dynamique", on entend la faculté que présentent ces agrafes ou plaques de générer une force de compression résultante entre les deux points au niveau desquels elles sont implantées et, plus particulièrement de part et d'autre d'un foyer de fracture osseux.

Les agrages chirurgicales utilisées pour la fixation des os et des tissus mous doivent posséder plusieurs caractéristiques essentielles. Tout d'abord, elles doivent développer une compression constante dans le temps. En outre, elles doivent être ancrées parfaitement, de façon à éviter le décrochement après leur implantation, décrochement généralement dû à des mouvements de l'articulation ou simplement de l'os sur lequel elles sont implantées. Enfin, le mode d'implantation ou d'enlèvement des agrafes et plaques doit être simple, facile à mettre en oeuvre, et générant un minimum de traumatismes osseux.

A ce jour, on connait différents types d'agrafes ou de plaques visant à atteindre ces buts. On a par exemple proposé, pour assurer leur rétention dans l'os mou, des agrafes dont les branches latérales sont munies de parties saillantes ou d'arêtes, destinées à empêcher le retrait de l'agrafe hors du tissu (FR-A-2 525 102). Néanmoins, si certes ce type d'agrafes annule tout risque de désancrage hors de l'os à maintenir, il ne génère aucune compression dynamique, et en outre, l'enlèvement de l'agrafe est générateur d'importants traumatismes osseux.

On a également proposé, notamment dans le document DE-A-2 703 529, de réaliser des agrafes en matériau martensitiques (en alliage de type Ni-Ti ou Ti-Nb), et de conférer aux branches des agrafes une mémoire de forme, susceptible d'induire un rapprochement de leur extrémité au-dessus de la température austénitique dudit matériau martensitique qui la compose. ce phénomène de "mémoire de forme" est dû à la transformation martensitique thermoélastique réversible. Ce phénomène, bien connu, consiste à donner à un matériau une forme définie que l'on traite à une température supérieure à la température austénitique As du matériau, puis à lui donner une autre forme également définie, à une température inférieure à la température martensitique Ms dudit matériau, et enfin à répéter plusieurs fois cette opération en fonction de la nature de l'alliage utilisé, afin de donner à ce matériau sa mémoire de forme définitive. Cette température Ms est inférieure à la température As.

Néanmoins, si certes avec ce type d'agrafes on obtient une compression dynamique au niveau de l'extrémité des agrafes, celle-ci s'avère généralement insuffisante pour la totalité de la fracture au niveau de laquelle est implantée l'agrafe, et même parfois rédhibitoire car cette compression est dissymétrique, en effet, elle rapproche les parties profondes de la zone de fracture et écarte la partie superficielle de cette même zone.

L'invention vise à pallier ces différents inconvénients. Elle vise une agrafe ou une plaque d'ostéosynthèse susceptibles de répondre aux différents impératifs énoncés ci-dessus, et aptes à générer une compression dynamique globale, au niveau de la fracture proprement dite sur laquelle est implantée l'agrafe ou la plaque, et présentant en outre des qualités d'auto-rétention.

Cette plaque d'ostéosynthèse est réalisée en alliage martensitique thermo-élastique, dont les températures de transformation sont :

– température de transformation martensitique Ms inférieure à 10° C ;

– température de transformation austénitique As supérieure à 15° C.

le passage de la température martensitique à la température austénitique induisant un raccourcissement de la longueur de la plaque.

Elle se caractérise en ce qu'elle est éduquée pour se présenter sous forme rectiligne à une température inférieure à la température de transformation martensitique Ms, et sous forme ondulée à une température supérieure à la température de transformation austénitique As.

L'invention concerne également une agrage d'ostéosynthèse présentant deux branches destinées à être insérées de part et d'autre du foyer de la fracture de l'os à réparer, lesdites branches étant éduquées pour se déformer et notamment se rapprocher sous l'effet de la température, au-dessus de la température de transformation austénitique As, lesdites branches étant reliées par une base de raccordement, constituée par une plaque du type de celle précédemment mentionnée.

De la sorte, lorsque l'ensemble de l'agrafe est à une température supérieure ou égale à la température de transformation austénitique, non seulement l'extrémité des agrafes se rapproche mais en outre et surtout, la base de raccordement elle-même se raccourcit, induisant au niveau de la fracture sur laquelle elle est implantée, une compression dynamique tant au niveau de l'os spongieux qu'au niveau de l'os cortical, ou au niveau des deux parties haute et basse de l'os cortical lorsque l'agrafe traverse l'os de part en part.

Avantageusement en pratique :

– l'agrafe est une agrafe monobloc ;

– les branches sont rapportées sur la base de raccordement ;

– seule une portion de la base de raccordement est éduquée ;

– la portion de la base réduite et éduquée est de section inférieure à la section totale de la base ;

– la portion ou la base de raccordement présente au moins deux orifices traversants situés au voisinage de leur extrémité, et destinés à permettre la fixation de ladite plaque ou base par tout moyen approprié, et notamment par des vis ;

– les extrémités libres des branches latérales de l'agrafe sont également éduquées pour augmenter leur surface dans le plan général les contenant à une température supérieure à la température austénitique ;

– l'une des branches latérales est constituée d'au moins trois sections, respectivement une première et une troisième sections, éduquées pour être globalement perpendiculaires à la base de raccordement à une température inférieure à la température martensitique, la première section, contigüe à ladite base étant éduquée pour s'écarter de l'autre branche latérale à une température supérieure à la température austénitique, et la troisième section étant éduquée pour au contraire se rapprocher de l'autre branche latérale à une température supérieure à la température austénitique, lesdites première et troisième sections étant en outre raccordées par une deuxième section, globlament parallèle à la base de raccordement ;

– l'extrémité libre de la troisième section est fendue longitudinalement, les deux zones ainsi définies étant éduquées pour rester parallèles et dans le prolongement de ladite troisième section à une température inférieure à la température martensitique, et pour s'écarter dans le plan de ladite section à une température supérieure à la température austénitique ;

– l'alliage de l'agrafe est un alliage à base de titane-nickel ou alliage cuivre-aluminium-zinc.

La manière dont l'invention peut être réalisée et les avantages qui en découlent ressortiront mieux des exemples de réalisation qui suivent, donnés à titre indicatif et non limitatif à l'appui des figures annexées dans lesquelles :

– la figure 1 est une vue schématique de l'agrafe conformément à l'invention à une température inférieure à la température de transformation martensitique ;

– la figure 2 est une vue similaire à celle de la figure 1 à une température supérieure à la température de transformation austénitique ;

– la figure 3 est une vue d'une plaque d'ostéosynthèse conforme à l'invention à une température inférieure à la température de transformation martensitique ;

– la figure 4 est une vue similaire à celle de la figure 3 à une température supérieure à la température de transformation austénitique ;

– la figure 5 est une vue d'une autre forme de réalisation de l'agrafe, à une température inférieure à la température martensitique ;

– la figure 6 est une vue similaire à celle de la figure 5, à une température supérieure à la température austénitique ;

– la figure 7 est une représentation d'une autre forme de réalisation de l'agrafe, à une température inférieure à la température martensitique ;

– la figure 8 est une vue similaire à celle de la figure 7, à une température supérieure à la température austénitique ;

– la figure 9 est une représentation d'une autre forme de réalisation de l'agrafe, à une température inférieure à la température martensitique ;

– la figure 10 est une vue similaire à celle de la figure 9, à une température supérieure à la température austénitique.

Une agrafe d'ostéosynthèse est fondamentalement constituée d'une base (1) sur laquelle sont rapportées les deux branches latérales (2,3). Ces branches latérales (2,3) sont lisses, ou granuleuses, et peuvent être légèrement effilées à leur extrémité libre (7,8), afin de faciliter leur insertion lors de l'implantation de l'agrafe à la température martensitique, au niveau des tissus osseux de la fracture. De manière connue, et selon les applications thérapeutiques particulières, la longueur des branches (2,3) peut être identique voire différente.

Selon une caractéristique fondamentale de l'invention, l'agrafe est réalisée en un matériau martensitique thermo-élastique, répondant aux critères de biocompatibilité requis. Typiquement, ce matériau martenstique est constitué par un alliage nickel-titane ou un alliage à base de cuivre, d'aluminium et de zinc.

La température martensitique Ms du matériau est typiquement voisine de 10°C. A cette température, les branches latérales (2,3) d'une part, et la base de raccordement (1) d'autre part, subissent des déformations répétées afin d'induire un effet de mémoire de forme, qui sera restitué lors du franchissement du seuil de température austénitique à savoir typiquement 25°C. Cette mémoire de forme peut être acquise par les différents éléments, à savoir branches latérales et base de raccordement en leur donnant une forme particulière à une température supérieure à la température de transformation austénitique As, puis en leur donnant une autre forme et notamment une forme droite à une température inférieure à la température de transformation martensitique. En répétant un certain nombre de fois ces transformations mécanique, une mémoire de forme rectiligne, respectivement pour la base de raccordement et les branches latérales à une température inférieure à la température martensitique, et une mémoire de forme ondulée, telle que représentée sur la figure 2 pour la base de raccordement avec les branches latérales se rapprochant pour une température supérieure au seuil austénitique, sont obtenues.

Dans une forme de réalisation particulière, l'agrafe est monobloc. Néanmoins, dans une autre forme de réalisation, on peut concevoir que les branches latérales (2,3) soient rapportées, et ce par tout moyen connu, sur la base de raccordement (1).

Dans une forme de réalisation représentée sur les figures 5 et 6, la base de raccordement est constituée de trois parties, à savoir deux parties extrêmes (4,5) de profil donné, et une partie intermédiaire (6) de section inférieure, joignant ces deux parties. De la sorte, on peut ainsi donner une mémoire de forme uniquement à cette partie intermédiaire, mémoire de forme ondulée, telle que représentée à la figure 6. Cette ondulation de la section intermédiaire (6) peut s'effectuer dans le plan général de la base de raccordement (1), tel que représenté dans la figure 6, ou dans une autre plan, et notamment dans un plan perpendiculaire au plan général de la dite base (1). Cette ondulation de la section intermédiaire (6) induit un raccourcissement de la base de raccordement (1) de l'agrafe, et par voie de conséquence une compression dynamique de la fracture à ce niveau. On peut observer sur les figures 3, 4, 5 et 6, que la longueur $l$ de la section de jonction (6) lorsqu'elle est rectiligne, c'est à dire, à une température inférieure à la température de transformation martensitique Ms, se réduit à la longueur $l' < l$ à une température supérieure à la température de transformation austénitique. Cette réduction entraîne la réduction de la longueur totale $L$ de la base de raccordement à une valeur $L' < L$ cette réduction ayant une valeur typique voisine de un millimètre et demi (1,5 mm).

Lorsque la base de raccordement (1) atteint une certaine longueur, il peut être nécessaire de solidariser celle-ci à l'os à réparer. On réalise cette solidarisation au moyen de vis, que l'on insère dans l'os au travers d'origices traversants (9,10) ménagés dans celle-ci au voisinage de ses extrémités (voir figure 3 - 6).

En d'autres termes, l'agrafe conforme à l'invention permet d'obtenir un double effet de compression et ce tant auniveau intramédulaire, par l'intermédiaire des branches latérales (2,3) dont les extrémités (7,8) se rapprochent, qu'au niveau externe à la surface même de l'os, au niveau de l'os cortical. En outre, compte-tenu de la déformation des extrémités des branches latérales de l'agrafe, celle-ci est auto-rétentive.

Pour sa mise en place, on porte l'ensemble de l'agrafe à une température inférieure à la température de transformation martensitique. A cette température, la base de raccordement (1) et les deux branches latérales (2,3) sont rendues rectilignes, lesdites branches étant globalement perpendiculaires à la base de raccordement (1). On implante alors l'agrafe en position rectiligne de part et d'autre du foyer de la fracture, et ce par compaction, des pré-trous ayant été préablement réalisés par le chirurgien. La température du corps humain étant supérieure à la température austénitique, l'agrafe se déforme et adopte une forme définie par la mémoire de forme déjà acquise. On observe d'une part, un raccourcissement de sa base de raccordement, et d'autre part, un rapprochement des extrémités libres (7,8) de ses branches latérales (2,3), conduisant outre à un double effet de compression déjà mentionné, à un résultat d'auto-rétention de l'agrafe.

Ainsi, aussi longtemps que la température de l'agrafe reste supérieure à la température austénitique, l'agrafe reste solidement implantée au niveau de l'os. Pour ôter l'agrafe, il suffit de refroidir l'agrafe à une température inférieure à la température de transformation martensitique, et ce par tout moyen quelconque. On peut ainsi retirer l'agrafe en douceur, sans risque de générer des traumatismes osseux.

On a représenté dans les figures 3 et 4, une plaque d'ostéosynthèse, d'un type tout à fait similaire à la base de raccordement ci-dessus. Cette plaque, comporte notamment des orifices traversants (9,10), destinés à permettre sa fixation au moyen de vis sur l'os à réparer. Comme dans le cas précédent, à une température supéireure à la température austénitique, la section intermédiaire se réduit d'une longueur $l$ à une longueur $l' < l$ induisant ainsi au niveau de l'os à réparer une compression dynamique.

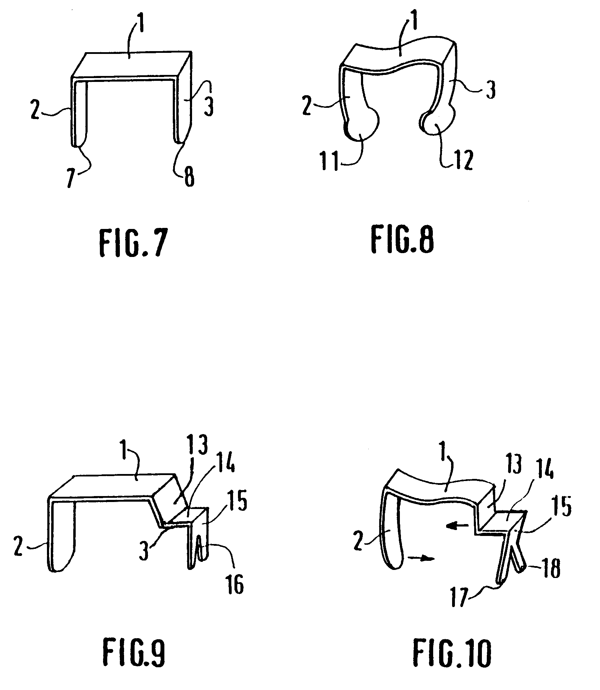

Dans une forme de réalisation plus particulièrement décrite dans les figures 7 et 8, l'extrémité libre (7,8) de chacune des branches latérales de l'agrafe est éduquée pour augmenter sa surface dans le plan contenant chacune des dites branches (figure 8), à une température supérieure à la température austénitique. Cet élargissement (11,12) permet ainsi d'augmenter la surface d'appui desdites extrémités au niveau de l'os spongieux, et ainsi d'améliorer la compression dynamique "interne" à l'os à réparer.

Dans une autre forme de réalisation décrite en liaison avec les figures 9 et 10, plus particulièrement adaptée au genou, l'une des branches latérales (3) est constituée de trois sections (13,14,15), chacune d'entre elles étant rectiligne à une température inférieure à la température de transformation martensitique. A une telle température, la première section (13) est légèrement écartée par éducation, de la verticale, et ce d'une valeur voisine de 15 degrés. En revanche, la direction de la troisième section (15) est globalement perpendiculaire à la base de raccordement (1) de l'agrafe. Elles sont reliées entre elles par une deuxième section (14), sensiblement parallèle à la dite base (1). En outre, la troisième section (15) présente à son extrémité une fente longitudinale (16) s'étendant sur quelques millimètres, typiquement dix millimètres.

La première section (13) reçoit une éducation de mémoire de forme telle, qu'à une température supérieure à la température austénitique, elle se rapproche de l'autre branche latérale (2), et devient globalement perpendiculaire à la base de raccordement (1), induisant par voie de conséquence, un rapprochement des autres sections (14,15) de ladite branche (2).

La troisième section (15) reçoit également une éducation de mémoire de forme, et ce à deux niveaux. Tout d'abord, à une température supérieure à la température austénitique, ladite troisième section (15) se rapproche de la branche latérale (2), et ce afin d'induire un effet de compression dynamique, similaire à celui décrit dans les exemples précédents. Ensuite, à une telle température, les deux zones (17,18) définies par la fente (16) au niveau de l'extrémité (8) de cette section s'écartent l'une de l'autre dans le plan de ladite section (15), afin d'induire un effet de rétention à ce niveau.

On peut également prévoir, pour cette forme de réalisation, de ménager des orifices traversants (non représentés) d'une part sur la base de raccordement (1), et d'autre part, sur la deuxième section (14) de la branche latérale, afin de permettre une fixation plus importante de l'agrafe sur son lieu d'implantation.

Les plaques et agrafes d'ostéosynthèse conformes à l'invention, se révèlent parfaitement adaptées aux rôles qui leur sont assignés, et notamment, à générer un double effet de compression dynamique au niveau des fracture osseuses.

## Revendications

1/ Plaque d'ostéosynthèse, réalisée en un alliage martensitique thermo-élastique, dont les températures de transformation sont :
   – température de transformation martensitique Ms inférieure à 10°C ;
   – température de transformation austénitique As supérieure à 15°C ;
le passage de la température martensitique à la température austénitique induisant un raccourcissement de la longueur de la plaque,
**caractérisée** en ce qu'elle est éduquée pour se présenter sous forme rectiligne à une température inférieure à la température de transformation martensitique Ms du matériau qui la compose, et sous forme ondulée à une température supérieure à la température de transformation austénitique As dudit matériau.

2/ Plaque d'ostéosynthèse selon la revendication 1, caractérisée en ce qu'elle comporte au voisinage de chacune de ses extrémités un orifice traversant (9,10), destiné à permettre la solidarisation de la plaque au moyen de vis dans l'os à réparer.

3/ Plaque d'ostéosynthèse selon l'une des revendications 1 et 2, caractérisée en ce que seule une portion (6) de ladite plaque est éduquée.

4/ Plaque d'ostéosynthèse selon la revendication 3, caractérisée en ce que la portion éduquée (6) de la plaque est de section inférieure à la section totale de ladite plaque.

5/ Agrafe d'ostéosynthèse comportant deux branches (2,3), destinées à être insérées de part et d'autre du foyer de la fracture de l'os à réparer, lesdites branches (2,3) étant éduquées pour se déformer et notamment se rapprocher sous l'effet de la température, au-dessus de la température de transformation austénitique As, lesdites branches étant reliées par une base de raccordement (1), caractérisée en ce que ladite base est constituée par une plaque selon l'une des revendications 1 à 4.

6/ Agrafe d'ostéosynthèse selon la revendication 5, caractérisée en ce qu'elle est monobloc.

7/ Agrafe d'ostéosynthèse selon la revendication 5, caractérisée en ce que les branches (2,3) sont rapportées sur la base de raccordement (1).

8/ Agrafe d'ostéosynthèse selon l'une des revendications 5 à 7, caractérisée en ce que les extrémités libres (7,8) des branches latérales (2,3) de l'agrafe sont également éduquées pour augmenter leur surface dans le plan général les contenant à une température supérieure à la température austénitique.

9/ Agrafe d'ostéosynthèse selon l'une des revendications 5 à 7, caractérisée en ce que l'une des branches latérales (3) est constituée d'au moins trois sections, respectivement :
   – une première section (13), contigüe à la base de raccordement (1), éduquée pour s'écarter de l'autre branche latérale (2) à une température inférieure à la température martensitique, et pour se rapprocher de cette dite branche latérale (2) à une température supérieure à la température austénitique,
   – une troisième section (15), éduquée pour que sa direction soit globalement perpendiculaire à la base de raccordement (1) à une température inférieure à la température martensitique, et pour s'écarter de l'autre branche latérale (2) à une température supérieure à la température austénitique,
   lesdites première (13) et troisième section (15) étant en outre raccordées par une deuxième section (14), globalement parallèle à la base de raccordement (1).

10/ Agrafe d'ostéosynthèse selon la revendication (9), caractérisée en ce que l'extrémité libre (8) de la troisième section (15) est fendue (16) longitudinalement, les deux zones (17,18) ainsi définies étant éduquées pour rester parallèles et dans le prolongement de ladite troisième section (15) à une température inférieure à la température martensitique, et pour s'écarter dans le plan de ladite section à une température supérieure à la température austénitique.

FIG.1

FIG.2

FIG.3

FIG.4

FIG.5

FIG.6

FIG.7

FIG.8

FIG.9

FIG.10

Office européen
des brevets

**RAPPORT DE RECHERCHE EUROPEENNE**

Numero de la demande

EP    91 42 0379

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | SU-A-940 759 (SAPELKIN) | 1-4 | A61B17/064 |
| Y | * figures 1,2 * | 5-8 | A61B17/58 |
|  | --- | | |
| X | US-A-3 786 806 (JOHNSON) | 1-4 | |
|  | * colonne 3, ligne 28 - ligne 34; figure 3 * | | |
|  | --- | | |
| Y | EP-A-0 145 166 (RAYCHEM) | 5-8 | |
|  | * exemples 4,5 * | | |
|  | --- | | |
| A | US-A-4 485 816 (KRUMME) | 5-8 | |
|  | --- | | |
| D,A | DE-A-2 703 529 (KRUPP) | 1 | |
|  | * page 5, ligne 25 * | | |
|  | --- | | |
| A | TECHN.MITT.KRUPP vol. 37, no. 1, 1979, pages 21 - 33; BENSMANN ET AL: 'UNTERSUCHUNGEN DER MEMORY-LEGIERUNG NICKEL-TITAN' * figure 2 * | 1 | |
|  | --- | | **DOMAINES TECHNIQUES RECHERCHES (Int. Cl.5)** |
| A | CA-A-1 149 106 (QUEEN'S UNIVERSITY) | | |
|  | ----- | | A61B A61F |

Le présent rapport a été établi pour toutes les revendications

| Lieu de la recherche | Date d'achèvement de la recherche | Examinateur |
|---|---|---|
| LA HAYE | 10 FEVRIER 1992 | BARTON S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P0402)